# DEMANDE DE BREVET EUROPEEN

(11) **EP 1 004 314 A1**
(43) Date de publication de la demande: **31.05.2000**
(21) Numéro de dépôt: 98122373.8
(22) Date de dépôt: 26.11.1998
(51) Int. Cl.: A61K 39/295

(54) **Vaccin T.d. Polio rappel pour une population vaccinée ou sensibilisée**

(71) Demandeur: Pasteur Merieux MSD, 69007 Lyon (FR)
(72) Inventeur: Cartier, Jean René, 69005 Lyon (FR); Laroche, Patrick, 78120 Rambouillet (FR)
(74) Mandataire: Gros, Florent

(57) **Abrégé**

Un vaccin formulé pour être utilisable exclusivement comme rappel vaccinal chez une population déjà primo-vaccinée ou sensibilisée contre au moins le poliovirus, *Corynebacterium diphteriae* et/ou *Clostridium tetani*, ledit vaccin comprenant : moins de 1,2 mg par ml de sel d'aluminium ; des antigènes immunogènes provenant au moins du poliovirus, de *Corynebacterium diphteriae* et de *Clostridium tetani* ; et une quantité d'anatoxine diphtérique utilisée comme antigène immunogène de *Corynebacterium diphteriae* comprise entre 4-16 Lf par ml. Utilisation d'antigènes immunogènes provenant au moins du poliovirus, de *Corynebacterium diphteriae* et de *Clostridium tetani*, pour la préparation d'un vaccin formulé pour être utilisable exclusivement comme rappel vaccinal chez une population déjà primo-vaccinée ou sensibilisée contre le poliovirus, *Corynebacterium diphteriae* et/ou *Clostridium tetani*, ledit vaccin étant destiné à minimiser les effets réactogènes et/ou allergiques induits par ces antigènes.Kit pharmaceutique comprenant au moins 2 doses injectables d'un vaccin.

## Description

La présente invention concerne un nouveau vaccin multivalent contre au moins la diphtérie, la poliomyélite et le tétanos, destiné à être utilisé comme rappel vaccinal chez une personne déjà primo-vaccinée ou sensibilisée.

### État de la technique

La diphtérie est due à *Corynebacterium diphteriae*. La transmission est essentiellement directe par les voies respiratoires. Les manifestations cliniques de la forme respiratoire relèvent de deux mécanismes. La prolifération des bactéries au niveau de la porte d'entrée détermine la symptomatologie locale, habituellement pharyngée (angine pseudo-membraneuse), parfois laryngée (croup). La diffusion de l'exotoxine de certaines souches est responsable d'angine maligne et/ou de complications viscérales (polynévrite, myocardite, syndrome malin de Marfan). De nombreuses études sérologiques, réalisées récemment dans la plupart des pays développés, ont montré que, selon l'âge, le sexe et l'histoire épidémique ou vaccinale locale, 20 à 80 % des habitants ne sont plus aujourd'hui correctement immunisés; et que les sujets de plus de 50 ans et les sujets féminins sont particulièrement vulnérables (Rappuoli *et al*., Vaccine 11, 576-577, 1993. Simonsen *et al*., Acta Pathol. Microbiol. Immunol. Scand, 95, 225-231, 1987. Wirz *et al*., Vaccine, 13, 771-773, 1995). Cette vulnérabilité est révélée ces vingt dernières années par la résurgence, parfois dramatique de la diphtérie dans les pays développés (Galazka *et al*., Eur. J. Epidemiol. 11, 107-117, 1995).

Le tétanos est dû à *Clostridium tetani*. La forme sporulée, essentiellement tellurique, introduite par effraction dans l'organisme (blessure, piqûre, brûlure ou plaie mineure), donne naissance à la forme végétative. Cette forme sécrète une endotoxine, la tétanospasmine. La lyse bactérienne provoque sa diffusion. Neurotrope, la toxine détermine la symptomatologie, c'est-à-dire un trismus inaugural et des contractures musculaires. Sans traitement, l'évolution est fatale par asphyxie ou syncope. La vaccination par anatoxine reste la seule parade. L'immunité acquise par primo-vaccination décroît avec le temps et nécessite des rappels. L'absence ou la perte avec l'âge de l'immunité a été notée dans la population américaine depuis des décennies par plusieurs enquêtes sérologiques (Hilton *et al*., Ann. Intern. Med., 115, 32-33, 1991). En Europe, des enquêtes sérologiques ont donné des résultats équivalents (Kjeldsen *et al.*, Scand. J. Infect. Dis., 20, 177-185, 1988). Il est donc clair que de nombreux adultes, particulièrement les plus de 50 ans et les femmes, sont redevenus ou ont toujours été vulnérables au tétanos et nécessitent une vaccination par anatoxine.

La poliomyélite est due au poliovirus, appartenant au groupe des entérovirus. Il existe trois sérotypes, dénommés type 1, type 2 et type 3. La transmission est essentiellement fécale-orale, directe ou indirecte, et parfois orale-orale. Le virus est doué d'un tropisme intestinal, musculaire, méningé et nerveux. L'infection est, en général inapparente ou fruste. Cette "poliomyélite-infection" est immunisante, mais sans immunité croisée entre les types, et contagieuse pendant plusieurs semaines. Elle suscite exceptionnellement (1 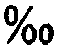 à 1 % des infections selon l'âge et le type) des formes méningées ou paralytiques (paralysie flasque aiguë). Cette "poliomyélitemaladie" est toujours grave car il n'existe pas de traitement curatif étiologique. Lorsqu'elle n'est pas mortelle par asphyxie (2 à 10 % selon l'âge et le type), elle est suivie de séquelles musculaires ou ventilatoires invalidantes. Les échanges humains, toujours plus nombreux, entre les pays d'endémie et les pays indemnes de poliomyélite, exposent ces derniers aux risques d'importation et de ré-introduction de souches sauvages. Ces dernières années, en dépit d'un niveau élevé de couverture vaccinale et d'une très faible incidence globale de la maladie, des cas importés et des épidémies sont apparues en Europe, en Amérique du Nord et au Moyen-Orient (OMS, Relevé Epidémiologique Hebdomadaire, 29, 220-221, 1996). En attendant une élimination de la maladie, les nombreux échanges humains entre les pays d'endémie et les pays indemnes risquent donc de provoquer dans n'importe quelle région du monde des flambées dues à des souches sauvages importées. A fin de réduire au minimum la propagation de la maladie lors de poussées de ce type, il importe que tous les pays, y compris ceux où plus aucun cas de poliomyélite aiguë n'est signalé, maintiennent un taux élevé de couverture vaccinale dans toute leur population.

Mis au point par Ramon dans les années 1930, les vaccins diphtérique et tétanique sont à base d'anatoxines. Elles peuvent être obtenues par l'action détoxifiante du formaldéhyde sur un concentré de culture de *Corynebacterium diphteriae* ou de *Clostridium tetani*, puis purification. Leurs activités immunisantes respectives sont appréciées *in vitro* et *in vivo*. Un test de floculation mesure la quantité d'anatoxine, exprimée en unité de floculation (Lf) par dose (Lyng *et al*., J. Biol. Stand., 15, 27-37, 1987 ; J. Lyng, Biologicals, 18, 11-17, 1990)

L'anatoxine tétanique est parfois administrée seule. Cependant, cette anatoxine est historiquement toujours combinée, au moins à l'anatoxine diphtérique. En général, la primo-vaccination tétanique et diphtérique est effectuée au cours de la première année de vie en 3 doses (OMS, WHO/EP/GEN, 95.3, 1995). Selon les pays, une dose de rappel est administrée au cours de la deuxième année et/ou entre 4 et 10 ans. Parfois, un rappel est également effectué entre 11 et 18 ans. En outre, l'OMS recommande, depuis 1987, de vacciner par anatoxine tétanique les femmes en âge de procréer dans les pays en développement, par 3 doses primo-vaccinales puis 1 dose de rappel 1 et 5 ans après.

La quantité d'anatoxine tétanique (T) par dose vaccinale varie selon les pays de 5 à 20 Lf pour une dose de 0,5 ml en primo-vaccination ou en rappel. La Pharmacopée Européenne recommande une activité d'au moins 20 UI.

La quantité d'anatoxine diphtérique (D) par dose vaccinale la plus largement et la plus anciennement administrée est celle qui existe dans les combinaisons primo-vaccinales pédiatriques : elle varie de 12 à 50 Lf pour une dose de 0,5 ml (Galazka *et al*., Vaccine, 14, 845-857, 1996).

Pour un rappel vaccinal, la réduction de la quantité d'anatoxine diphtérique (d) s'est maintenant aussi généralisée. La quantité est généralement de l'ordre de 1/10 de la quantité pédiatrique. Elle est ainsi fixée à 2 Lf pour une dose de 0,5 ml aux US et au Canada, et est recommandée en rappel à partir de l'âge de 7 ans (Edsall *et al*., Am. J. Hyg, 53, 283-295, 1951). En Europe, la quantité d'anatoxine et l'âge inférieur d'administration changent selon les pays. La quantité n'est pas fixée pour la raison que la seule condition imposée par la Pharmacopée Européenne porte non pas sur la quantité mais sur l'activité elle doit être en moyenne de 2 UI par dose de 0,5 ml.

Pour les vaccins de rappel destinés à une population adulte, l'anatoxine diphtérique est généralement aussi combinée à l'anatoxine tétanique. Il est connu que cette combinaison réduit quelque peu la tolérance de l'anatoxine tétanique seule (Palmer *et al*., Br. Med. J., 286, 624-626, 1983). Aux Etats-Unis, les rapports d'évènements indésirables liés aux vaccins T et Td administrés chez les sujets de plus de 7 ans ont été récemment analysés (Haber *et al*., ICAAC, 1996). Le vaccin Td a exposé davantage que le vaccin T à une réaction locale, une dyspnée, une perte de connaissance ou une convulsion.

Pour ce qui concerne la poliomyélite, deux vaccins ont été mis au point dans les années 1950 : le vaccin injectable (VPI), inactivé au formaldéhyde, développé par Salk (Plotkin *et al*., E.A. Vaccines, ed. Raven Press, 1994), et le vaccin oral (VPO), vivant atténué, développé par Sabin (Plotkin et al., *supra*). Le VPI a permis à tous les pays d'éliminer la poliomyélite, tant sauvage que postvaccinale (Murdin et al., Vaccine, 14, 735-746, 1996). Le mode de production et la composition du VPI varie selon les pays. Les trois types de poliovirus sont ainsi cultivés sur lignée cellulaire continue VERO, puis purifiés et inactivés par le formaldéhyde. Leur activité immunisante respective est appréciée *in vitro* et *in vivo*. Un ELISA mesure la teneur en antigène viral, exprimée en unités internationales: l'OMS recommande par dose de 0,5 ml 40 UI, 8 UI et 32 UI respectivement pour les types 1, 2 et 3.

Le VPI offre deux avantages pharmaceutiques : il est stable et ne nécessite pas de logistique particulière. Il peut être parfois combiné à d'autres antigènes.

La vaccination simultanée pendant l'enfance contre la poliomyélite, le tétanos et la diphtérie est une pratique courante depuis des années dans le monde entier, voire depuis des décennies dans la plupart des pays développés. Cette pratique a fortement contribué à la réduction considérable du nombre de cas et de morts dus à ces trois maladies. Cependant, elles n'ont pas disparu. Dans les pays développés, lorsqu'elles surviennent, elles sont d'autant plus graves qu'elles sont plus tardives. Or l'immunité acquise par vaccination s'atténue avec le temps. Puisqu'ils ne bénéficient pas d'un entretien naturel de cette immunité, les adolescents et les adultes de ces pays redeviennent vulnérables. Le maintien de la couverture vaccinale contre chacune des trois maladies tout au long de la vie est désormais une obligation épidémiologique.

Actuellement, il n'existe aucune combinaison vaccinale contre au moins la diphtérie, le tétanos et la poliomyélite, prévu spécialement pour un rappel vaccinal chez l'adulte ou l'adolescent, qui viserait à prolonger une protection conférée initialement lors d'une primo-vaccination ou une sensibilisation, et qui minimiserait sur cette population les effets indésirables induits par les vaccins existants.

Les vaccins pédiatriques injectables du genre TDPolio (T: anatoxine tétanique ; D : dose classique d'anatoxine diphtérique ; Polio : poliovirus inactivés types 1, 2 et 3), par exemple le vaccin D.T.Polio® (PMsv S.A., France) ne peuvent satisfaire ce besoin. En effet, ces types de vaccin sont utilisés principalement en primo-vaccination pédiatrique. Il sont dépourvus de sels d'aluminium. Ils contiennent des quantités trop importantes d'anatoxine diphtérique, de l'ordre de 100 Lf /ml, par exemple. Et ils déclenchent des réactions indésirables chez l'adulte (Björkholm *et al*., Eur. J. Clin. Microb., 6, 637-640, 1987).

On ne peut aussi envisager de simplement modifier un vaccin TDPolio existant afin d'en minimiser les effets indésirables sur une population primo-vaccinée. En effet, une cible de choix serait par exemple d'y réduire la quantité d'anatoxine diphtérique. Dans ce cas, on n'envisagerait pas de retenir les quantités d'anatoxine utilisées classiquement dans un vaccin Td (qui est la seule référence actuelle), de l'ordre d'au moins 4 Lf par ml, par exemple, mais plutôt dans mettre plus, probablement plus de 20 Lf/ml, par exemple. En effet, dans un vaccin Td classique l'anatoxine diphtérique est toujours adjuvantée par un sel d'aluminium, ce qui renforce son immunogénicité. Dans un vaccin TDPolio, le fait de ne pas avoir d'adjuvant implique de renforcer la dose d'anatoxine par rapport à celle utilisée dans un vaccin Td.

Pour les mêmes raisons, la simple combinaison d'un vaccin existant du genre Td injectable, par exemple les vaccins vaccinol® ou Td-Pur® (Chiron-Behring GmBH, Allemagne) ou le vaccin Diftavax® (PMsv S.A., France), avec un vaccin classique contre la poliomyélite (PMsv, France), ne pourrait, non plus, conduire à une solution satisfaisante. En effet, le choix de chaque constituant dans un tel vaccin, ainsi que leur dosage, sont déterminants pour l'obtention d'une réponse immunitaire optimale, et une minimisation des effets indésirables.

Ainsi par exemple si l'on rajoute de nouveaux antigènes (Polio) à un vaccin Td, la quantité de sels d'aluminium est alors susceptible d'être insuffisante pour jouer un rôle adjuvant optimal. L'immunogénicité d'un tel vaccin risquerait d'être ainsi réduite. Par contre, si l'on veut pallier ce problème en augmentant la quantité de sels d'aluminium, on risquerait parallèlement d'aggraver les réactions indésirables liées à ces sels (1,2 à 3 mg par ml dans les vaccins Td; Gupta *et al*., Vaccine, 13, 1263-1276, 1995).

De même, si l'on rajoute de nouveaux antigènes (Polio) à un vaccin Td, on diminue aussi la charge relative de chaque antigène T ou d. L'immunogénicité d'un tel vaccin risquerait d'être ainsi aussi réduite. Par contre, si l'on veut pallier ce problème en augmentant le dosage de chaque antigène, notamment celui de l'anatoxine diphtérique, on risquerait parrallèllement d'aggraver les réactions indésirables liées à cette anatoxine (Björkholm *et al*., Eur. J. Clin. Microb., 6, 637-640, 1987).

Le choix de chaque constituant d'un vaccin du type TdPolio, ainsi que leur dosage, sont donc difficiles à cerner, et ne peuvent être déduit aisément des vaccins existants.

Il n'existe pas non plus une combinaison vaccinale, utilisable exclusivement lors d'un rappel vaccinal chez l'adulte ou l'adolescent, contre la diphtérie, le tétanos, la poliomyélite et la coqueluche, voire même aussi contre l'hépatite A et/ou l'hépatite B.

La présente invention vise ainsi à fournir un vaccin ayant au moins la combinaison de base TdPolio, utilisable exclusivement pour un rappel vaccinal chez une population primo-vaccinée ou sensibilisée qui, tout en se distinguant des vaccins TDPolio, Td et Polio précédents, présente une immunogénicité comparable à ceux-ci et en plus minimise leurs effets indésirables.

### Résumé de l'invention

A cet effet, l'invention concerne un vaccin formulé pour être utilisable exclusivement comme rappel vaccinal chez une population déjà primo-vaccinée ou sensibilisée contre au moins le poliovirus, *Corynebacterium diphteriae* et/ou *Clostridium tetani*, ledit vaccin comprenant:
- moins de 1,2 mg par ml de sel d'aluminium,
- des antigènes immunogènes provenant au moins du poliovirus, de *Corynebacterium diphteriae* et de *Clostridium tetani*, et
- une quantité d'anatoxine diphtérique utilisée comme antigène immunogène de *Corynebacterium diphteriae* comprise entre 4-16 Lf par ml.

L'invention concerne aussi une utilisation d'antigènes immunogènes provenant au moins du poliovirus, de *Corynebacterium diphteriae* et de *Clostridium tetani,* pour la préparation d'un vaccin formulé pour être utilisable exclusivement comme rappel vaccinal chez une population déjà primo-vaccinée ou sensibilisée contre au moins l'un de ces antigènes, ledit vaccin étant destiné à minimiser les effets réactogènes et/ou allergiques induits par ces antigènes.

Enfin l'invention concerne un kit pharmaceutique comprenant au moins 2 doses injectables d'un vaccin selon la présente invention.

### Description détaillée de l'invention

Dans le contexte de la présente invention, on entend par l'expression 〈〈 une population déjà primo-vaccinée ou sensibilisée 〉〉, des personnes adultes, adolescentes ou juvéniles ayant déjà été vaccinées contre au moins le poliovirus, *Corynebacterium diphteriae* et/ou *Clostridium tetani*, la population préférée étant constituée par les adolescents et les adultes, et plus particulièrement les personnes agées.

La présente invention concerne donc un vaccin utilisable exclusivement pour un rappel vaccinal chez une population déjà primo-vaccinée ou sensibilisée, comprenant un ou plusieurs antigènes immunogènes provenant au moins du poliovirus, de *Corynebacterium diphteriae* et de *Clostridium tetani*.

De préférence, les antigènes de *Corynebacterium diphteriae* et de *Clostridium tetani* ont pour origine leurs toxines, qui sont détoxifiées par le formaldéhyde puis purifiées. Les techniques de détoxification et de purification de ces toxines sont bien connues depuis des décennies et sont incorporées par référence dans la description de la présente invention, telles que celles décrites par Leong *et al.*, Science, 220, 815-517, 1983; Ramon G., Ann. Inst. Pasteur, 38, 1-105, 1924; Raynaud *et al*., Ann. Inst. Pasteur, 96, 60-71, 1959 ; ou par Bizzini *et al* ., Eur. J. Biochem., 17, 100-105, 1970, par exemple. Les analogues détoxifiés pouvant être produits par génie génétique sont également compris dans la présente invention.

De même, les antigènes du poliovirus peuvent être constitués simplement par un ou plusieurs types de poliovirus inactivé (voir ci-après) et/ou par des antigènes immunogènes purifiés du poliovirus tels que ceux décrits par Delpeyroux *et al*., 70, 1065-73, 1988 ; EP323861 (Pasteur Institut) ; EP86707 (Pasteur Institut) ; ou dans EP65924 (Pasteur Institut), par exemple.

Pour obtenir des poliovirus inactivés, on peut les cultiver sur des lignées cellulaires VERO, les purifier, puis les détoxifier avec du formaldéhyde, par exemple. Les techniques de culture et de détoxification du poliovirus sont bien connues depuis des décennies et sont aussi incorporées par référence dans la description de la présente invention, telles que celles décrites dans WO9800167 (Connaught); Dulbecco, Nature, 376, p.216, 1995 ; Cohen, Acta Leiden, 56, 65-83, 1987, et par Salk, Dev Biol Stand., 47, 247-55, 1981, par exemple.

De préférence, on utilise les poliovirus inactivés types 1 (souche MAHONEY), 2 (souche MEF 1) et 3 (souche Saukett).

La quantité d'anatoxine diphtérique utilisée par dose vaccinale comme antigène immunogène de *Corynebacterium diphteriae* doit être comprise entre 4 à 16 Lf par ml, de préférence 6-14 Lf, notamment de l'ordre de 10 Lf, par exemple. Cette quantité permet d'assurer une immunogénicité optimale, tout en minimisant les effets indésirables, comme les réactions réactogènes ou allergiques aux antigènes, par exemple.

De même la quantité d'anatoxine tétanique par dose vaccinale comme antigène immunogène de *Clostridum tetani* est comprise entre 6 à 30 Lf par ml, de préférence 8-20 Lf, notamment de l'ordre de 10 Lf, par exemple. Cette quantité permet d'assurer une immunogénicité optimale, tout en minimisant les effets indésirables.

Pour mesurer la quantité d'anatoxine diphtérique ou tétanique (Lf), on utilise le test de floculation décrit par Lyng (J. Biol. Stand., 15, 27-37, 1987 ; ou Biologicals, 18, 11-17, 1990) ou l'OMS (Manual for the production and the control of vaccines, BLG/UNDP/77.1 et BLG/UNDP/77.2), en prenant en compte toutefois que l'on rapporte la pureté de l'anatoxine utilisée à 1500-1800 Lf par mg d'azote pour la diphtérie, ou 1000-1300 Lf par mg d'azote pour le tétanos, avant de mesurer une quelconque quantité dans une dose vaccinale.

Ainsi, une dose vaccinale pour un rappel anti-dipthérie, tétanos et poliomyélite comprenant une quantité d'anatoxine diphtérique inférieure ou supérieure au domaine 4-16 Lf par ml, peut être quant même couverte par la présente invention, dans la mesure où l'on tombe toujours dans la fourchette de 4-16 Lf par ml si l'on rapporte la pureté de l'anatoxine purifiée à 1500-1800 Lf par mg d'azote. La connaissance de la pureté de l'anatoxine diphtérique utilisée est donc déterminante. A titre d'indication, dans la plupart des productions, la pureté de l'anatoxine dipthérique purifiée est généralement de l'ordre de 1700-1800 Lf par mg d'azote (source interne). De même, pour l'anatoxine tétanique, cette pureté est de l'ordre de 1300-1400 Lf (source interne).

En ce qui concerne les poliovirus inactivés utilisés comme antigènes immunogènes, une dose vaccinale selon l'invention peut comprendre 60-120 UI/ml d'antigène D du poliovirus type 1, 8-30 UI/ml d'antigène D du poliovirus type 2, et/ou 16-80 UI/ml d'antigène D du poliovirus type 3, par exempte. Les Unités Internationales sont déterminées selon la méthode Sigmoïde.

Dans un mode d'application particulier de la présente invention, le vaccin selon l'invention peut comprendre, en outre de la combinaison de base TdPolio décrite ci-dessus, 0,1 à 60 µg / mi de l'anatoxine de *Bordetella pertussis*, 0,1 à 40 µg /ml de l'antigène HBs de l'hépatite B, et/ou 0,1 à 40 µg/ml de virus hépatite A inactivé ou un antigène de celui-ci, par exemple.

La toxine de *Bordetella pertussis*, y compris les analogues détoxifiés produits par génie génétique, peut être produite de différentes manières. Par exemple, on peut cultiver une souche de *B. pertussis* selon des méthodes traditionnelles (Sekura *et al*., J.Biol. Chem., 258, 14647-14651, 1993), on peut isoler l'anatoxine en adsorbant le milieu de culture sur une colonne Affi-Gel Blue® (Bio-Rad Lab, US), puis en l'éluant avec une solution riche en sels (par exemple 0,75 M de chlorure de magnésium), puis après avoir ôter les sels, on peut encore ensuite adsorber cet éluat sur une colonne d'affinité fetuin-Sépharose (composé de fetuin lié à du bromure de cyanogène) puis l'éluer avec une solution 4M d'un sel de magnésium. La toxine de *B. pertussis* peut être ensuite détoxifiée à l'aide du glutaraldéhyde selon une méthode modifiée de Munoz *et al*. (Infect. Immun., 33, 820-826, 1981) comme décrit dans la demande de brevet PCT/EP97/05378 (PMsv). De nombreuses autres méthodes sont encore à la disposition de l'homme du métier, comme celles de Irons *et al*. (Biochem. Biophys. Acta, 580, 175-185, 1979), ou celles décrites dans les brevets US4705686 et EP336736, et sont incorporées par référence dans la description de la présente invention.

On peut produire une suspension de l'antigène de l'hépatite B selon la méthode décrite dans le brevet EP273811 (Pasteur Vaccins), dans laquelle on produit des particules antigéniques de surface de l'hépatite B par expression à partir d'une culture de cellules CHO tranfectées par un plasmide ponant le gène HBsAg de façon à libérer les particules de surface antigéniques dans le milieu de culture. D'autres techniques sont bien connues de l'homme du métier et sont incorporées par référence dans la description de la présente invention, telles que celles décrites dans EP864649, UE56711 ou IE48665, par exemple.

De même on peut produire un virus de l'hépatite A inactivé selon le protocole de Flehmig *et al*. (Viral Hepatitis and Liver Disease, 87-90, 1988; J. Med.Virol., 22, 7-16, 1987). D'autres techniques sont bien connues de l'homme du métier et sont incorporées par référence dans la description de la présente invention, telles que celles décrites par Wang *et al*., Vaccine, 13, 835-40, 1995 ; Shevtsova *et al*., Zh Mikrobiol Epidemiol Immunobiol., 2, 55-90, 1995 ; Richtmann *et al*., J Med Virol., 48, 147-50, 1996 EP199480 ; IE48399 ; ou dans IE50191, par exemple..

Le vaccin selon l'invention comprend un ou plusieurs adjuvants choisis parmi les adjuvants reconnus comme tels, notamment tous les sels d'aluminium, comme les phosphates et hydroxydes d'aluminium; l'adjuvant de Freund; le N-acétylmuramyl-L-alanyl-D-isoglutamyl-L-alanine-2-[1,2-dipalmitoyl-sn-glycéro-3-(hydroxyphosphoryloxy)] (voir Sanchez-Pescador *et al*., J. Immu., 141, 1720-1727, 1988) ; les molécules dérivées de *Quillaja saponaria*, comme le Stimulon® (Aquila, US) ; l'Iscoms® (CSL ltd, US) ; toutes molécules à base cholestérol et analogues, comme le DC Chol® (Targeted Genetics); le glycolipide Bay R1005® (Bayers, DE) ; les antigènes de *Leishmania brasiliensis* comme le LeIF (nom technique) disponible auprès de Corixa Corp. (US), les polymères de la famille de polyphosphazènes, comme l'Adjumer (nom technique) disponible auprès du 〈〈 Virus Research Institute 〉〉 (US), par exemple.

On peut remarquer que, par rapport aux vaccins TDPolio classique, comme le D.T. Polio® (PMsv, France), la présente invention propose pour la première fois l'adjonction d'un adjuvant, et notamment un sel d'aluminium, comme l'hydroxyde d'aluminium, par exemple.

Contre toute attente, le vaccin selon la présente invention peut comprendre une quantité de sel d'aluminium plus faible que celles rencontrées dans tous les vaccins Td classiques, comme le Td-Pur® ou Diftavax®, alors que l'on aurait pu estimer nécessaire d'en augmenter la charge suite à l'adjonction des poliovirus inactivés. Un vaccin selon la présente invention peut ainsi comprendre moins de 1,2 mg/ml d'un sel d'aluminium, de préférence moins de 0,8 ou 0,7 mg/ml, par exemple.

Un vaccin selon la présente invention peut comprendre d'autres constituants, comme des agents conservateurs tels que le 2-phénoxyéthanol et/ou le formaldéhyde, des traces d'antibiotiques, etc., par exemple.

Le vaccin selon l'invention peut se présenter sous la forme d'une suspension injectable, faiblement opalescente du fait de la présennce d'un sel d'aluminium insoluble. La dose vaccinale est de préférence de l'ordre de 0,5 ml, contenue dans une seringue en verre pré-remplie. L'administration est effectuée de préférence par vois intramusculaire, par exemple dans un des muscles deltoïde.

L'invention concerne aussi une utilisation d'une combinaison d'antigènes immunogènes provenant au moins du poliovirus, de *Corynebacterium diphteriae* et de *Clostridium tetani* pour la préparation d'un vaccin, formulé pour être utilisable exclusivement comme rappel vaccinal chez l'adulte et l'adolescent déjà primo-vaccinés et/ou sensibilisés à au moins l'un de ces antigènes. Les différentes formulations retenues peuvent être l'une de celles décrites ci-dessus, par exemple, notamments celles comprenant d'autres antigènes provenant de *Bordetella pertussis*, de l'hépatite A ou de l'hépatite B.

Dans un mode d'application particulier de la présente utilisation, plusieurs doses de vaccin sont destinées à être injectées séparément à un même sujet dans un intervalle de temps compris entre 10 jours à 3 mois, de sorte à favoriser une réaction immunitaire optimale, et de sorte à minimiser les effets réactogènes et/ou allergiques de ces antigènes.

Le vaccin selon l'invention permet de diminuer les effets indésirables induits par les vaccins TDPolio et Td existants lorsqu'ils sont injectées à des sujets adultes ou adolescents. On peut ainsi observer une diminution des réactions locales comme un engourdissement des membres, des sueurs, de la fièvre, des douleurs associés à des rougeurs, des nodules, indurations et/ou ecchymoses, et une diminution des dyspnées, pertes de connaissance ou convulsions, par exemple. Ces réactions peuvent être classées parmi les réactions allergiques, voire réactogènes, aux antigènes et/ou à un certains autres composés du vaccin, tel que le sel d'aluminium.

La présente invention est décrite plus en détail dans les exemples présentés ci-après. Les pourcentages sont donnés en poids sauf indication contraire. Il va de soi, toutefois, que ces exemples sont données à titre d'illustration de l'objet de l'invention dont ils ne constituent en aucune manière une limitation.

### Exemple 1 Immunogénicité du TdPolio avec différentes doses d'anatoxine diphtérique

On prépare de l'anatoxine diphtérique (d) en cultivant la souche IM 1514 N3S dans un milieu IMD pendant 15 à 18 h à 36°C, en centrifugeant le milieu, en le clarifiant, en le concentrant par ultrafiltration, en le détoxifiant à 37°C pendant 4 semaines en présence de 6/1000 de formaline, puis en purifiant la toxine jusqu'à atteindre une pureté de l'ordre de 1700 Lf par mg d'azote.

Parallèlement, on prépare de l'anatoxine tétanique (T) en cultivant la souche Harvard N^{o}49205 IM 1472C dans un milieu Massachusetts à 35°C pendant 4 jours, en y ajoutant du NaCl et citrate de sodium, en centrifugeant le milieu, en le concentrant par ultrafiltration, en le détoxifiant pendant 2 semaines à 35°C en présence de 5,5/1000 de formaline et 5 g/l de bicarbonate de sodium, puis en purifiant la toxine jusqu'à atteindre une pureté de l'ordre de 1200 Lf par mg d'azote.

De même, on prépare les poliovirus inactivés types 1 (souche MAHONEY), 2 (souche MEF 1) et 3 (souche Saukett), selon la méthode Salk.

On mélange ensuite les 5 antigènes immunogènes ci-dessus avec de l'hydroxyde d'aluminium, du 2-phénoxyéthanol, du formaldéhyde et de l'eau. Pour ce faire, on stérilise le gel d'aluminium en présence d'eau, on ajuste le pH entre 5,6 et 6, on ajoute séquentiellement l'ADP et l'ATP, on ajuste le pH à 6-6,9, on ajoute les trois types de poliovirus, puis on ajoute le 2-phénoxyéthanol et le formaldéhyde.

Le produit final de 0,5 ml servira de vaccin TdPolio, et contient au minimum 2 UI d'anatoxine diphtérique, au minimum 20 UI d'anatoxine tétanique purifiée. 40 UI, 8 UI et 32 UI d'antigène D respectivement pour les poliovirus inactivés du type 1, 2 et 3, 0,35 mg d'hydroxyde d'aluminium, 2,5 µl de 2-phénoxyéthanol, 12,5 µg de formaldéhyde ; et le reste d'eau.

Une étude de phase I, a eu comme principal objectif de valider la tolérance clinique et biologique de la première administration d'un vaccin TdPolio adsorbé. Dans ce but, 31 adultes volontaires sains ont été recrutés. Trois lots du vaccin de 0,5 ml chacun ont été utilisés. Ils ne différaient que par la quantité d'anatoxine diphtérique purifiée décrite ci-dessus: 2Lf, 5Lf et 8 Lf. Ils ont été attribués séquentiellement à raison de 10 sujets par lot. Chaque sujet a été vacciné par une dose d'un lot injectée dans un muscle deltoïde.

Aucune réaction générale n'a été rapportée dans les groupes vaccinés par les lots à 2, 5 et 8 Lf. Au moins une réaction locale a été rapportée au cours de la première semaine chez 8 sujets du groupe vacciné par le lot à 2 Lf d'ADP, chez 6 sujets du groupe vacciné par le lot à 5 Lf d'ADP et chez 8 sujets du groupe vacciné par le lot à 8 Lf d'ADP, il s'agissait toujours de douleurs, associées à quelques rougeurs, nodules, indurations et/ou ecchymoses. Toutes les réactions ont disparu sans traitement et n'ont pas modifié la vie courante des sujets. Aucune réaction n'est survenue au-delà de la première semaine. Aucun événement indésirable grave n'a été déclaré par les investigateurs.

Cette étude a eu aussi comme objectif secondaire d'évaluer l'immunogénicité des trois premiers lots du vaccin adsorbé. Les résultats montrent que la réponse immunitaire aux cinq antigènes a été excellente pour les trois lots. En dépit de titres initiaux élevés dus au jeune âge des sujets et à des vaccinations récentes, un effet rappel a été obtenu pour chaque antigène.

### Exemple 2 Immunogénicité chez le jeune adulte

L'immunogenicité et l'innocuité d'un vaccin TdPolio a été déterminée pendant un essai clinique chez 508 jeunes adultes.

Pour chaque sujet, une dose de 0,5 ml d'un vaccin Td (Diftawax®) ou TdPolio a été injecté dans le muscle deltoïde gauche, et une dose de 0,5 ml du vaccin contre la poliomyélite (VPI®, PMsv) ou un placebo a été injecté dans le muscle deltoïde droit. Le vaccin adsorbé Td contient de l'anatoxine tétanique (activité ≥20 IU), de l'anatoxine diphtérique (activité ≥2 IU) de l'hydroxyde d'aluminium (≤ 1,25 mg). Le vaccin VPI contient les antigènes des poliovirus du type 1 (40 UI), 2 (8 UI) et 3 (32 UI). Le placebo a la même composition que le VPI à la différence près qu'il ne contient pas d'antigènes D. Le vaccin TdPolio a la même composition que celles décrites à l'exemple 1 (5 Lf d'ADP).

Les anticorps anti-diphtérie, tétanos et poliomyélite sont déterminées à partir des sérums des sujets par test ELISA.

Les résultats montrent qu'avant vaccination quasiment tous les sujets étaient séropositifs en ce qui concerne l'ADP (99,2% ont un titre ≥ 0,01 UI/ml : protection possible; et 92,6% ont un titre ≥ 0.1 UI/ml : protection assurée). Un mois après la vaccination avec le TdPolio, 99,6% des sujets avaient un titre d'anticorps ≥ 0,1 UI/ml et 82,4% un titre ≥ 1 UI/ml (protection à long terme). Au total, 17,4% des sujets ont présenté une séroconversion.

De même, la majorité des sujets étaient séropositifs en ce qui concerne l'anatoxine tétanique (99,6% ayant un titre ≥ 0.01 UI/ml : protection possible ; et 98,4% ayant un titre ≥ 0.1 UI/ml : protection assurée). Un mois après la vaccination avec le TdPolio, 100% des sujets ont un titre en anticorps ≥ 0,1 UI/ml, et 99,2% ont un titre ≥ 1 UI/ml (protection à long terme). Au total, 25,2% des sujets ont présenté une séroconversion.

Pour ce qui concerne une protection contre la poliomyélite la majorité des sujets étaient séropositifs en ce qui concerne les trois types de poliovirus. Au total, 99,2% des sujets avaient un titre en anticorps ≥ 5 pour le type 1, 100% pour le type 2 et 97,6% pour le type 3. Un mois après la vaccination avec le TdPolio, 100% des sujets ont un titre ≥ 5 pour tous les types de poliovirus avec un titre minimum de 120 pour le type 1, 160 pour le type 2 et 80 pour le type 3. Au total, 63,1% des sujets ayant déjà un titre élevé en anticorps présentent une séroconversion.

Lorsque l'on compare les résultats obtenus avec le TDPolio et ceux obtenus en combinant les vaccins de référence Td et VPI®, on obtient une réponse en anticorps équivalente.

### Exemple 3 Innocuité et tolérabilité chez le jeune adulte

L'inocuité et la tolérabilité d'un vaccin TdPolio a été déterminée parmi 1742 jeunes adulte. Chacun de ces sujets a reçu une dose de 0,5 ml injecté dans le muscle deltoïde gauche du vaccin présenté à l'exemple 1 (ADP : 5 Lf). Les effets ont été évalués 15 minutes après vaccination. L'effet local, régional et systémique ont été évalués pendant le mois suivant la vaccination.

L'effet immédiat commun est l'apparition d'une rougeur au point d'injection (0,34% des sujets) et une douleur (0,11%).

Les effets communs locaux pendant le mois suivant la vaccination sont les suivants pour 66% des sujets: douleur (64,41%), rougeur au point d'injection (9,13%), et nodules sous-cutanés (3,33%) Ces effets indésirables apparaissent les 3 premiers jours suivant la vaccination et durent 2 à 3 jours. 0,86% des sujets ont aussi rapporté des événements d'oedème, d'inflammation, de migraine, d'engourdissement du bras, des contractions involontaires des muscles et une paraesthésie.

Les effets communs systémiques pendant le mois suivant la vaccination sont les suivants pour 18% des sujets: maux de tête (10,5%), nausées ou vomissements (2,75%), et malaises (2,41%). Aucun événement d'urticaire ou de démangeaison généralisé n'ont été rapportés. Ces événements apparaissent les 3 premiers jours suivants la vaccination et durent 2 à 3 jours. Seul 0,23% des sujets ont une température dépassant ≥ 40°C durant les 3 premiers jours suivant la vaccination..

### Exemple 4 Inocuité et tolérabilité du TdPolio versus Td + Polio chez les jeunes personnes

Le vaccin TdPolio décrit à l'exemple 2 montre une excellente tolérabilité au cours de l'essai sur 508 jeunes adultes (exemple 2). Les résultats sont d'ailleurs comparables à ceux obtenus au cours de l'essai décrit à l'exemple 3.

En faisant la soustraction du pourcentage de sujets ayant eu au moins un signe local ou régional au site d'injection du placébo (14,8%) de ceux observés au site d'injection du vaccin Polio (36,5%), puis en additionnant ce pourcentage à celui observé au site d'injections du vaccin Td (66,7%), on observe une différence d'environ 8% sur l'apparition d'évenements indésirables (88,4% vs 80,5%). Le vaccin TdPolio est donc mieux toléré que l'association des vaccins existants.

### Exemple 5 Immunogénicité chez les personnes au-delà de 40 ans

L'immunogénicité et l'inocuité d'un vaccin TdPolio a été déterminée pendant un essai clinique chez 113 sujets âgés au-delà de 40 ans (40 à 78 ans). Tous les sujets avaient reçu une primo-vaccination (3 doses pendant un an) contre la diphtérie, le tétanos et la poliomyélite, la dernière vaccination remontant respectivement à 32 ans (minimum 15 ans), 28 ans (minimum 10 ans) et 28 ans (minimum 10 ans).

Les sujets ont reçu une injection d'une dose de 0,5 ml du TdPolio décrit à l'exemple 1 (ADP 5Lf) dans le muscle deltoïde droit, et 28 jours plus tard une deuxième injection dans le muscle gauche. Les anticorps anti-diphtérie, tétanos et poliomyélite ont été évaluées à partir des sérums des sujets par test ELISA.

Les résultats montrent que seulement 50% des sujets étaient initialement protégés contre le tétanos, menant ainsi en évidence le déclin de l'immunité. 83% des sujets étaient initialement séropositifs contre les poliovirus types 1, 2 et 3. Au jour 28 suivant la première vaccination, la proportion de sujets protégés contre la diphtérie, le tétanos et le poliovirus types 1, 2 et 3 était montée à 80,5%, 97,3% et 100%. respectivement. Au jour 56 suivant l'administration de la seconde dose, la proportion de sujets protégés contre la diphtérie, le tétanos et le poliovirus types 1, 2 and 3 était montée à 93,7%, 100% et 100%, respectivement.

### Exemple 6 Inocuité et tolérabilité du TdPolio versus Td + Polio chez les personnes au-delà de 40 ans

L'inocuité et la tolérabilité du vaccin TdPolio décrit à l'exemple 5 a été évaluée. Les résultats montrent un profile de tolérabilité similaire à celui obtenu avec l'association Td + Polio. Le vaccin TdPolio induit cependant moins d'évenements indésirables sérieux (moins de 1,8 pour 1000 injections).

### Exemple 7 Vaccin rappel pour adulte d.T.Polio.AcP

On prépare une suspension vaccinale de 0,5 ml, avec ou sans conservateur, constitué de 5 Lf d'anatoxine diphtérique (ayant une pureté de 1700 Lf par mg d'azote), 5 Lf d'anatoxine tétanique (ayant une pureté de 1200 Lf par mg d'azote). 40 UI, 8 UI et 32 UI d'antigène D respectivement pour les poliovirus inactivés du type 1, 2 et 3 ; 6 µg/ml d'anatoxine purifiée (AcP) de *Bordetella pertussis*, 0,35 mg d'hydroxyde d'aluminium, et le reste d'eau.

On peut aussi ajouter comme conservateur 2,5 µl de 2-phénoxyéthanol et 12,5 µg de formaldéhyde.

Les anatoxines diphtérique et tétanique, ainsi que les poliovirus inactivés ont été préparées comme décrit à l'exempte 1.

L'anatoxine de *B. pertussis* est préparée selon la méthode de Sekura *et al* (J. Biol. Chem., 258, 14647-14651, 1993), détoxifiée selon un protocole modifié de Munoz *et al*. (Infect. Immun., 33, 820-826, 1981), puis préadsorbée sur un gel d'aluminium.

On mélange ensuite les antigènes immunogènes ci-dessus avec de l'hydroxyde d'aluminium, de l'eau, et le cas échéant avec les conservateurs. Pour ce faire, on stérilise le gel d'aluminium en présence d'eau, on ajuste le pH entre 5,6 et 6, on ajoute séquentiellement l'ADP, l'ATP et l'anatoxine de *B. pertussis*, on ajuste le pH à 6,8-7, on ajoute les trois types de poliovirus, puis le cas échéant on ajoute le 2-phénoxyéthanol et le formaldéhyde.

### Exemple 8 Vaccin rappel pour adulte d.T.Polio.AcP.HBs

On prépare une suspension vaccinale de 0,5 ml, sans conservateur, constitué de 5 Lf d'anatoxine diphtérique (ayant une pureté de 1700 Lf par mg d'azote), 5 Lf d'anatoxine tétanique (ayant une pureté de 1200 Lf par mg d'azote), 40 UI, 8 UI et 32 UI d'antigène D respectivement pour les poliovirus inactivés du type 1, 2 et 3 ; 6 µg/ml d'anatoxine purifiée (AcP) de *Bordetella pertussis*, 5 µg/ml d'antigène HBs de l'hépatite B, 0,35 mg d'hydroxyde d'aluminium, et le reste d'eau.

Les anatoxines diphtérique et tétanique, ainsi que les poliovirus inactivés ont été préparés comme décrit à l'exemple 1. L'anatoxine de *B. pertussis* a été préparée comme décrit à l'exemple 7.

L'antigène HBs a été préparé selon la méthode décrite dans le brevet EP 273811 (Pasteur Vaccins). Il est stabilisé par une préadsorbtion sur un gel d'aluminium.

On mélange ensuite les antigènes immunogènes ci-dessus avec de l'hydroxyde d'aluminium et de l'eau. Pour ce faire, on stérilise le gel d'aluminium en présence d'eau, on ajuste le pH entre 5,6 et 6, on ajoute séquentiellement l'ADP, l'ATP et l'anatoxine de *B. pertussis*, on ajuste le pH à 6,8-7, on ajoute les trois types de poliovirus, on ajuste le pH à 6,8 si nécessaire, et on ajoute l'HBs.

### Exemple 9 Vaccin rappel pour adulte d.T.Polio.AcP.HBs.HA

On prépare une suspension vaccinale de 0,5 ml, sans conservateur, constitué de 5 Lf d'anatoxine diphtérique (ayant une pureté de 1700 Lf par mg d'azote), 5 Lf d'anatoxine tétanique (ayant une pureté de 1200 Lf par mg d'azote), 40 UI, 8 UI et 32 UI d'antigène D respectivement pour les poliovirus inactivés du type 1, 2 et 3 ; 6 µg/ml d'anatoxine purifiée (AcP) de *Bordetella pertussis*, 5 µg/ml d'antigène HBs de l'hépatite B, 5 µg/ml de virus de l'hépatite A inactivé, 0,35 mg d'hydroxyde d'aluminium, et le reste d'eau.

Les anatoxines diphtérique et tétanique, ainsi que les poliovirus inactivés ont été préparés comme décrit à l'exemple 1. L'anatoxine de *B. pertussis* a été préparée comme décrit à l'exemple 7. L'antigène HBs a été préparé comme décrit à l'exemple 8.

Le virus inactivé de l'hépatite A est préparé selon la méthode de Flehmig *et al*. (*supra*).

On mélange ensuite les antigènes immunogènes ci-dessus avec de l'hydroxyde d'aluminium, les conservateurs et de l'eau. Pour ce faire, on stérilise le gel d'aluminium en présence d'eau, on ajuste le pH entre 5,6 et 6, on ajoute séquentiellement l'ADP, l'ATP et l'anatoxine de *B. pertussis*, on ajuste le pH à 6,8-7, on ajoute les trois types de poliovirus, on ajuste le pH à 6,8 si nécessaire, puis on ajoute séquentiellement l'HBs et le virus de l'hépatite A inactivé.

## Revendications

1. Un vaccin formulé pour être utilisable exclusivement comme rappel vaccinal chez une population déjà primo-vaccinée ou sensibilisée contre au moins le poliovirus, *Corynebacterium diphteriae* et/ou *Clostridium tetani*, ledit vaccin comprenant:
- moins de 1,2 mg par ml de sel d'aluminium,
- des antigènes immunogènes provenant au moins du poliovirus, de *Corynebacterium diphteriae* et de *Clostridium tetani*, et
- une quantité d'anatoxine diphtérique utilisée comme antigène immunogène de *Corynebacterium diphteriae* comprise entre 4-16 Lf par ml.

2. Un vaccin selon la revendication 1, caractérisé en ce que la quantité d'anatoxine diphtérique est comprise entre 6 à 14 Lf par ml.

3. Un vaccin selon la revendication 2, caractérisé en ce que ta quantité d'anatoxine diphtérique est de l'ordre de 10 Lf par ml.

4. Un vaccin selon la revendication 1 caractérisé en ce qu'il comprend en outre au moins un antigène choisi parmi les antigènes de *Bordetella pertussis*, de l'hépatite A ou de l'hépatite B.

5. Utilisation d'antigènes immunogènes provenant au moins du poliovirus, de *Corynebacterium diphteriae* et de *Clostridium tetani*, pour la préparation d'un vaccin formulé pour être utilisable exclusivement comme rappel vaccinal chez une population déjà primo-vaccinée ou sensibilisée contre le poliovirus, *Corynebacterium diphteriae* et/ou *Clostridium tetani*, ledit vaccin étant destiné à minimiser les effets réactogènes et/ou allergiques induits par ces antigènes.

6. Utilisation selon la revendication 5 pour la préparation d'un vaccin selon l'une des revendications 1 à 4, ledit vaccin étant destiné à minimiser les effets réactogènes et/ou allergiques induits par ces antigènes et/ou les sels d'aluminium.

7. Utilisation selon la revendication 5, pour la préparation d'au moins deux doses de vaccin destinées à être injectées séparément à un même sujet adulte dans un intervalle de temps compris entre 10 jours à 3 mois, ledit vaccin étant destiné à minimiser les effets réactogènes et/ou allergiques induits par ces antigènes.

8. Kit pharmaceutique comprenant au moins 2 doses injectables d'un vaccin selon l'une des revendications 1 à 4.
